(19) Europäisches Patentamt
European Patent Office
Office européen des brevets

(11) Publication number: 0 105 487 B1

(12) # EUROPEAN PATENT SPECIFICATION

(45) Date of publication of patent specification: 02.01.91

(21) Application number: 83109782.9

(22) Date of filing: 29.09.83

(51) Int. Cl.$^5$: C 07 D 317/20, C 07 D 319/06, C 07 C 43/178, C 07 C 49/84, C 07 C 317/22, C 08 G 65/38

(54) **Process for preparing polyacetal polymers and monomers employed therein.**

(30) Priority: 30.09.82 US 430358

(43) Date of publication of application: 18.04.84 Bulletin 84/16

(45) Publication of the grant of the patent: 02.01.91 Bulletin 91/01

(84) Designated Contracting States: AT BE CH DE FR GB IT LI LU NL SE

(56) References cited:
GB-A-1 493 759
US-A-3 734 888
US-A-4 175 175

CHEMICAL ABSTRACTS, vol. 94, no. 19, May 11, 1981, Columbus, Ohio, USA; T. OSHIMA et al. "Facile synthetic methods of acetals", page 617, columns 1, 2, abstract no.156469m

(73) Proprietor: AMOCO CORPORATION
200 East Randolph Drive P.O. Box 5910-A
Chicago Illinois 60680 (US)

(72) Inventor: Kelsey, Donald Ross
490 Auten Road 4B
Somerville New Jersey 08876 (US)

(74) Representative: Barz, Peter, Dr. et al
Patentanwälte Dr. V. Schmied-Kowarzik Dipl.-Ing. G. Dannenberg Dr. P. Weinhold Dr. D. Gudel Dipl.-Ing. S. Schubert Dr. P. Barz
Siegfriedstrasse 8
D-8000 München 40 (DE)

(56) References cited:
CHEMICAL ABSTRACTS, vol.91, no. 20, November 12, 1979, Columbus, Ohio, USA; M.K. AKKAPEDDI et al. "Cationic polymerization of 2,2-dimethyl-1,3-dioxole and 2,2-diphenyl-1,3-dioxole", page 5, column 2, abstract no. 158177b

CHEMICAL ABSTRACTS, vol. 91, no. 4, July 23, 1979, Columbus, Ohio, USA; B. YOON et al. "Chromophoric structures of alkali lignin", page 113, column 2, abstract no.22670z

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 105 487 B1

(56) References cited:
CHEMICAL ABSTRACTS, vol. 93, no. 8, August 25, 1980, Columbus, Ohio, USA; J.A. HEMMINGSON et al. "The self-condensation of the lignin model compounds, vanillyl and veratryl alcohol", page 130, column 2, abstract no. 74117k

CHEMICAL ABSTRACTS, vol. 86, no. 11, March 14, 1977, Columbus, Ohio, USA; W. TADROS et al. "Reactions with stilbenes and related derivatives", page 564, column 1, abstract no. 72057x

**Description**

Bisphenols are used for the preparation of a variety of useful polymeric materials, including epoxy resins, polyesters, polycarbonates, and especially polyarylethers as described in, for example, US—A—4,175,175. However, the monomers (acetalized bisphenols) of this invention are novel compounds.

The formation of the acetal moiety in a number of diverse organic compounds is well known in the art. The processes for the preparation of acetals and their corresponding heteroatom analogues, such as hemithioacetals, can be accomplished by a number of synthetic methods. Examples of such methods have been reviewed, for example, in the following: H. J. Lowenthal in "Protective Groups in Organic Chemistry," J. F. W. McOmie, ed., Plenum Press, 1973, p. 323ff; S. R. Sandler et al., "Organic Functional Group Preparations," Vol. 3, Academic Press, 1972, p. 2ff; C. A. Buehler et al., "Survey of Organic Synthesis," Wiley-Interscience, 1970, p. 513ff and vol. 2, 1977, p. 461ff; R. T. Bergstrom in "The Chemistry of Ethers, Crown Ethers, Hydroxyl Groups and Their Sulfur Analogues," Suppl. E, Part 2, S. Patai, ed., Wiley, 1980, p. 881ff; and F. A. J. Meskens, Synthesis, 501(1981); Houben Weyl, Sauerstoffverbindungen I/3 (1965), pages 221—224.

Commonly, the acetal group is formed by reacting the corresponding carbonyl group with an alcohol or an alcohol derivative such as an orthoester, generally in the presence of an acid catalyst. It is also advantageous to conduct the reaction under conditions so as to remove the water of reaction and byproducts, as for example, by distillation, by employing azeotropic solvents, as described in, for example M. Sulzbacher et al, J. D—13,434 Amer. Chem. Soc., 70, 2827 (1948), by using drying agents, or by adding chemical agents such as trialkyl orthoesters or tetraalkyl orthosilicates.

The use of clay catalysts to prepare acetals is known in the art as taught by Thuy et al., (Bull Soc. Chim. France, 2558 (1975)) and by Taylor et al., (Synthesis, 467 (1977)) both of which employed montmorillonite clay catalysts. However in the case of the heretofore unknown acetal bisphenol monomers of this invention, improved processes for the efficient preparation of said monomers are highly desirable.

US—A—3,734,888 is directed to polyacetals and a process for their preparation. The polyacetals are described in this patent as containing groups of the formula:

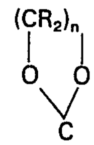

wherein the R is hydrogen or alkyl of 1 to 3 carbon atoms and n is 2 or 3. The polyacetals are prepared by reacting an aromatic polyketone and a diol of the formula $HO—(CR_2)_n—OH$ in the presence of an acid catalyst. The polyacetals in the reference are described as soluble in solvents, and solutions of the polyacetals in such solvents are useful as coating lacquers for metal conductors in wire or sheet form, for various plastic films, such as polyamides and polyesters and as adhesives. Additionally, the polyacetals are described as useful in film or powder form as adhesives in thermally bonding metals and plastics when melt pressed to such materials.

Described herein are novel monomers and a process for producing polyacetals from said monomers.

The monomers of this invention have not been previously described in the literature nor have they been used to prepare polymers, i.e. polyacetals as herein defined.

Although methods known in the art may be applicable to the preparation of the acetal monomers of this invention, improved processes are desirable for the preparation of said acetal monomers and especially, for the preparation of the acetals derived from glycols and diaromatic ketones having one or more hydroxyl groups situated ortho or para to the carbonyl.

The reaction of a glycol, an orthoester, and a solid catalyst, such as clay, with a diaromatic ketone produces the corresponding glycol acetal in improved yields and reaction times.

As compared to the process described in US—A—3,734,888, *supra*, wherein preformed aromatic polyketones are converted to the corresponding polyacetals under acid conditions, the process of this invention allows the preparation of polyketals from novel monomers under basic conditions.

The novel monomers of this invention are of the following formulae:

HO—K—OH

wherein K has up to 40 carbon atoms and is selected from one or more of the following:

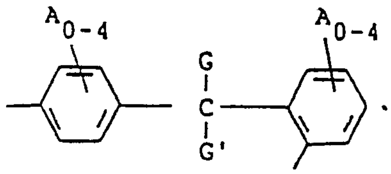
,
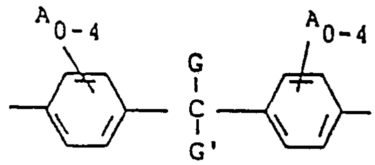

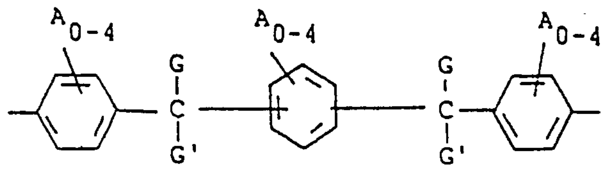

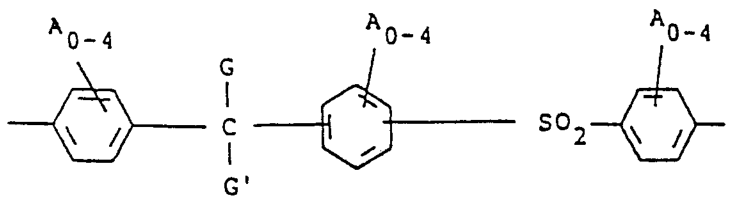

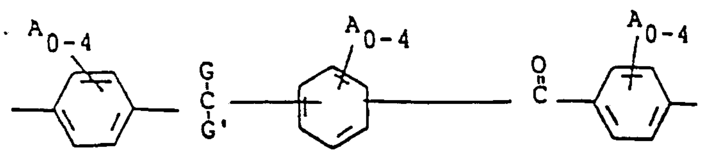

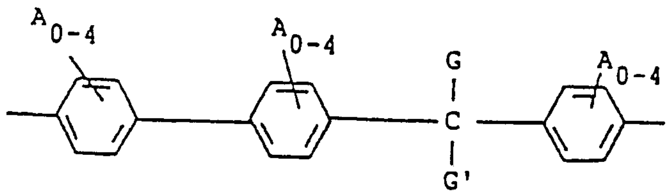

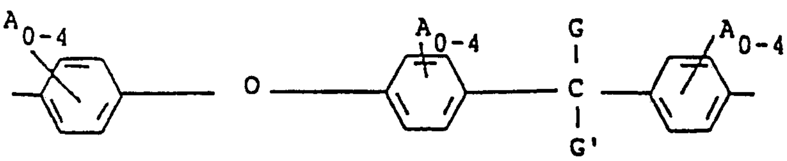

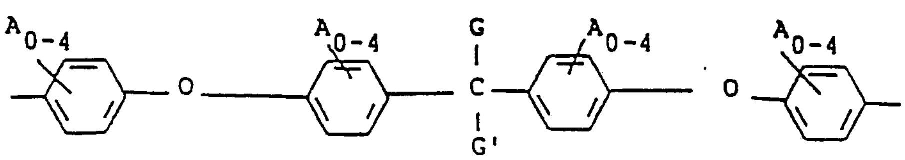

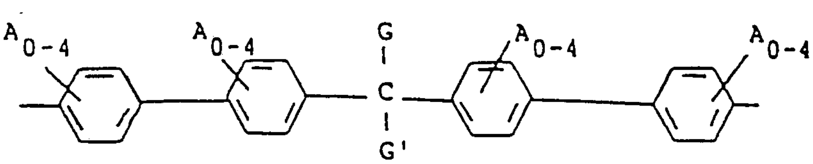

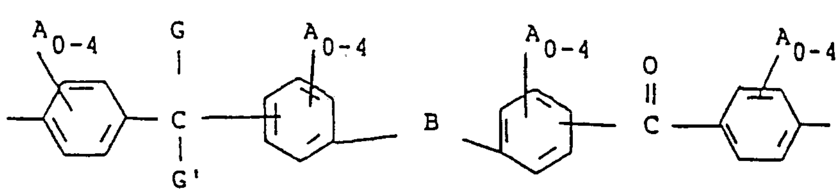

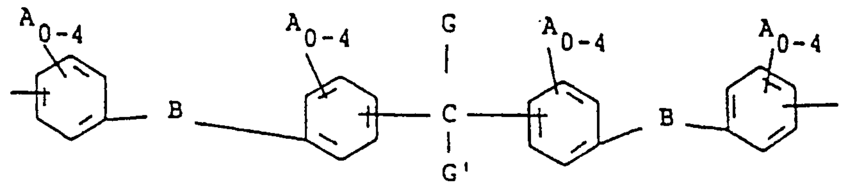

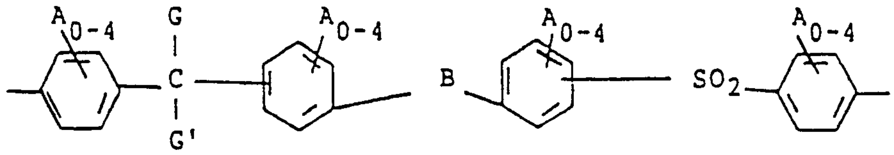

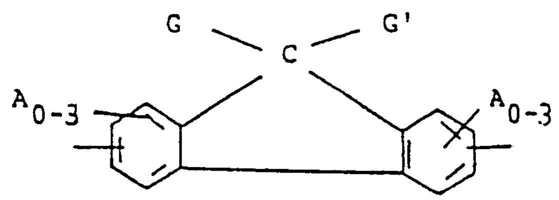

wherein A is independently selected from alkyl, aryl, halogen and cyano, which are non-interfering under the reaction conditions, B is an electron withdrawing group in ortho- or para-position, selected from —$SO_2$—, —CO—, —SO—, or a single bond, —O—, —S—, —S—S— or a difunctional hydrocarbon radical of from 1 to 20 carbon atoms and G and G′ may be the same or different and represent —OR, wherein the groups R are each independently alkyl, aryl or arylalkyl of from 1 to 20 carbon atoms, may be substituted or unsubstituted, may contain heteroatoms and may also be connected by a chemical bond thus connecting G and G′, with the proviso that the R's should not contain functionality which is base sensitive.

Preferred and most preferred monomers are described below. According to the present invention the polyacetal is prepared from the following monomers:

(a) one or more monomers HO—K—OH as defined above

(b) one or more monomers X—Z—Y, where Z is

$$-Ar^1-Q-\left[-Ar^2-Q'-\right]_n-Ar^3- \quad \text{or} \quad \overset{Q''}{\overset{|}{-Ar^4-}}$$

wherein The Ar's are substituted or unsubstituted aryl radicals of up to 18 carbon atoms, n is 0 to 3, Q and Q′ are electron withdrawing groups ortho or para to X and Y and selected from —$SO_2$—, —CO—, —SO—, and Q″ is an electron withdrawing group ortho or para to X and Y and selected from —$NO_2$, —CN, or —$SO_mR'$, R′ being a hydrocarbon group, and m being 1 or 2, and wherein X and Y are halogen (such as —F and —Cl), —OSOR′ or —$OSO_2R'$;

(c) optionally one or more bisphenols HO—W—OH, where W is selected from the following:

$$-Ar^1-Q-\left[-Ar^2-Q'-\right]_n-Ar^3-, \quad -Ar^4- \quad \text{and} \quad -Ar^5-V-Ar^6-,$$

where n, the Ar's, Q and Q′ are as defined above, and wherein V is a single bond, —O—, —S—, —S—S— or a difunctional hydrocarbon radical of from 1 to 20 carbon atoms, such as alkyl, aryl and alkylaryl radicals and rings fused to both $Ar^5$ and $Ar^6$.

Preferably, the polyacetal is derived from the following monomers of classes (b) and (c):

(b) one or more monomers X—Z—Y, where Z is selected from the following:

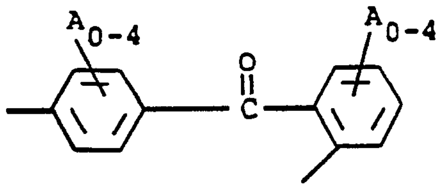 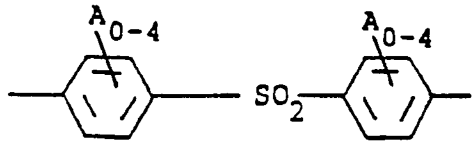 ,

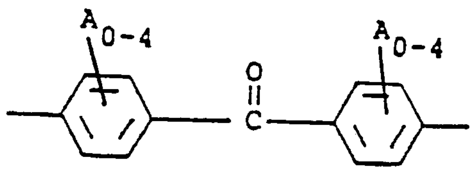 , 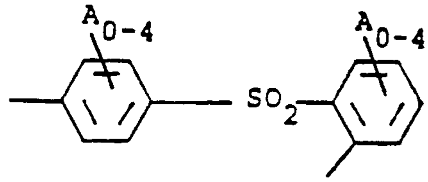 ,

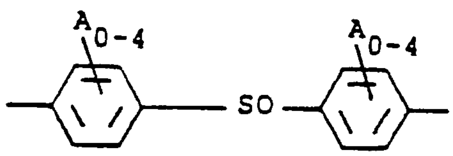 , 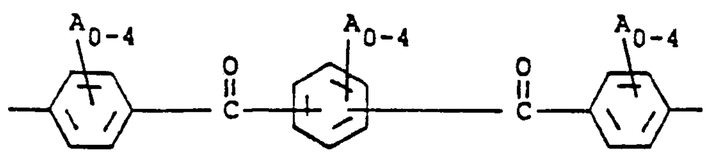 ,

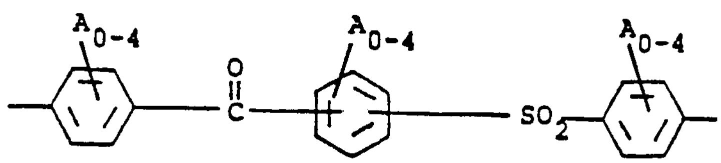 ,

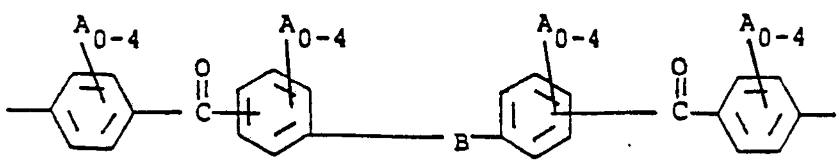 ,

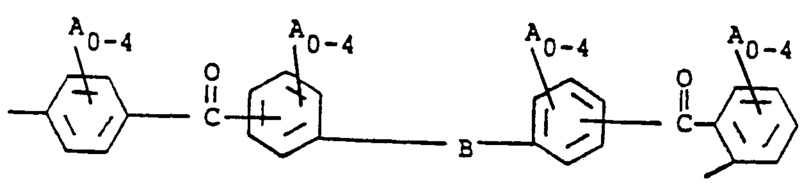 ,

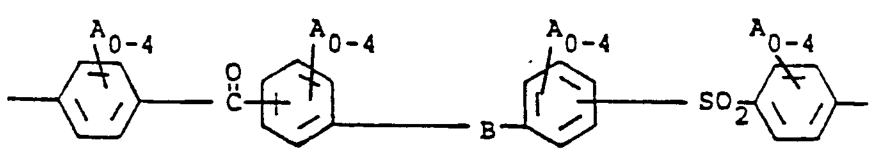 ,

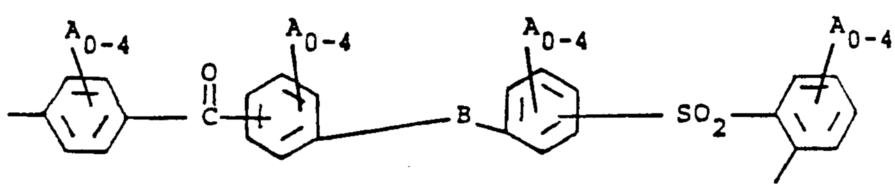 ,

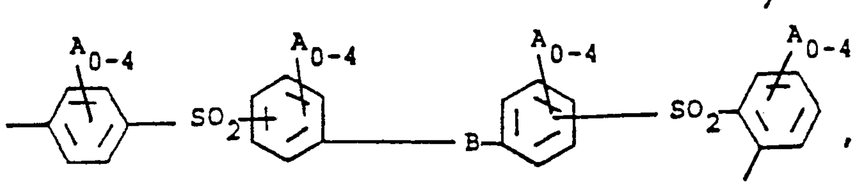 ,

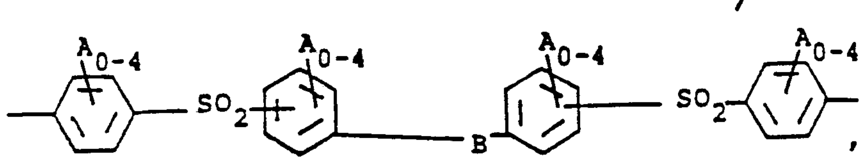 ,

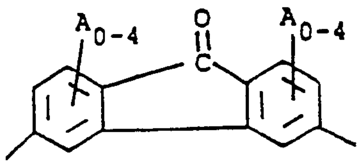,
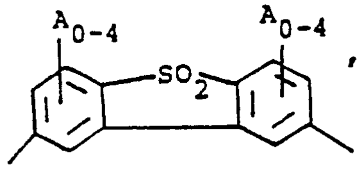,
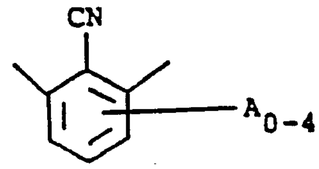

wherein B is defined as above for V and A is a non-interfering substituent group unreactive under the polymerization conditions and independently selected from alkyl, aryl, halogen, cyano, and wherein X and Y are halogen; most preferably Z is

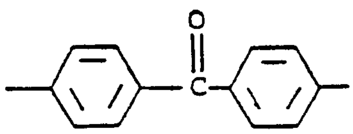 and 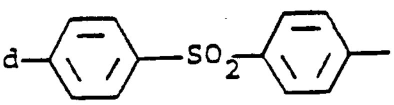

and X and Y are F or Cl;

(c) optionally one or more comonomer bisphenols HO—W—OH, where W is selected from the following:

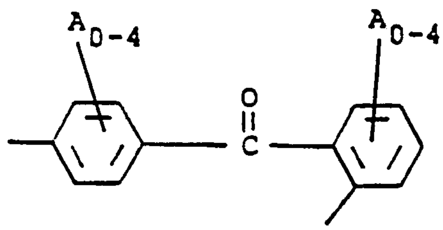
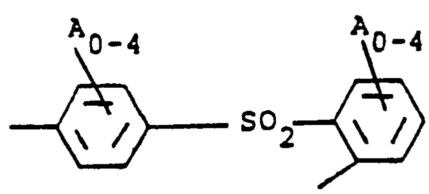
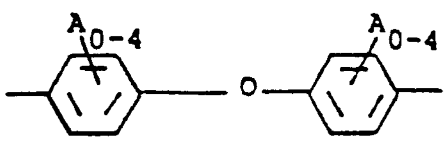
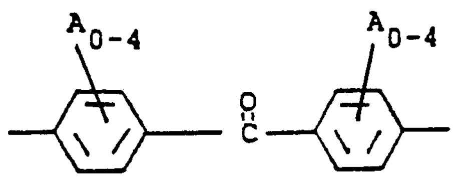
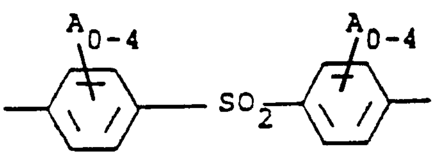
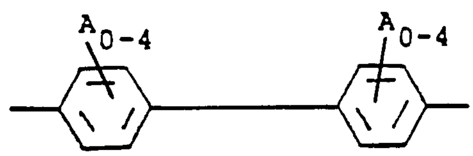
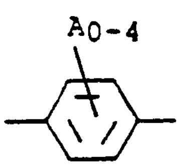,
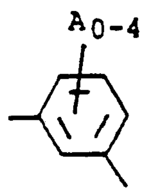
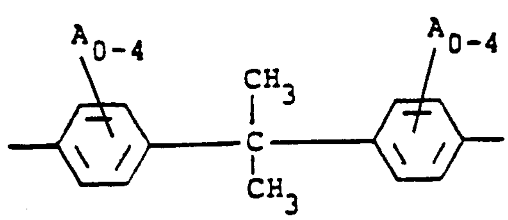
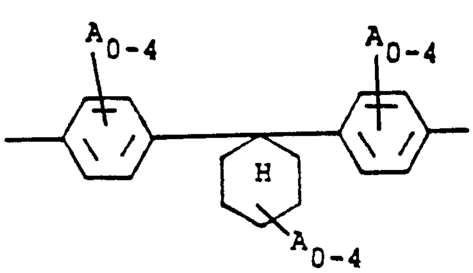
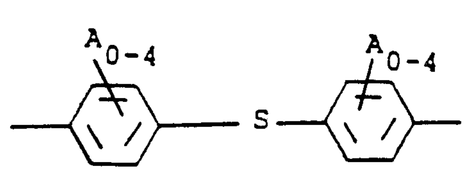

wherein A is defined as above; most preferably W is selected from the following:

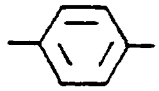 , 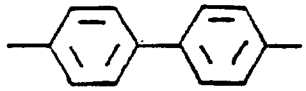 , 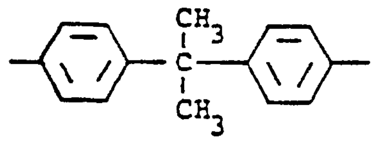

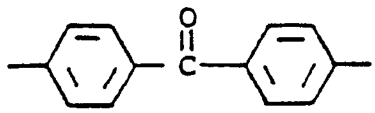 , or 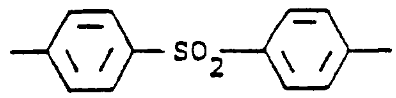

Examples of R in the groups —OR representing G and G′ in the formulae of the class (a) monomers include e.g. methyl, ethyl, propyl, isopropyl, benzyl, cyclohexyl, and where G and G′ are connected e.g.

$-CH_2CH_2-$, $-CH(CH_3)CH_2-$, $-CH(CH_3)CH(CH_3)-$, $-C(CH_3)_2CH_2-$, $-C(CH_3)_2CH(CH_3)-$,

$-C(CH_3)_2C(CH_3)_2-$, $-CH_2CH_2CH_2-$, $-CH_2C(CH_3)_2CH_2-$,

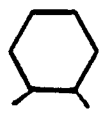 , 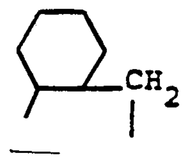 , 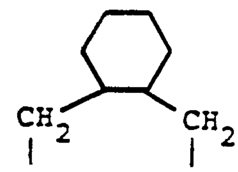

The preferred acetal bisphenol monomers are characterized as having formula (i) or (ii)

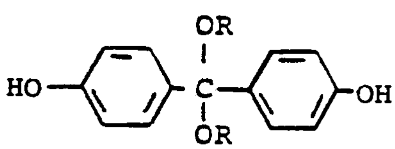

(i)

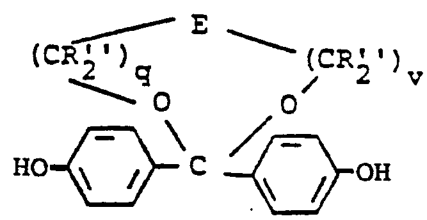

(ii)

where R is as defined above and R″ is independently selected from hydrogen, alkyl, aryl and arylalkyl containing from 1 to 20 carbon atoms, E is selected from a single bond, double bond, difunctional hydrocarbon, carbonyl, —O—, —S—, —SO—, $-SO_2-$, —NR—, and difunctional silicon, q is 1 or 2, and v is 1 or 2.

The most preferred acetal bisphenol monomers are of the following formulae:

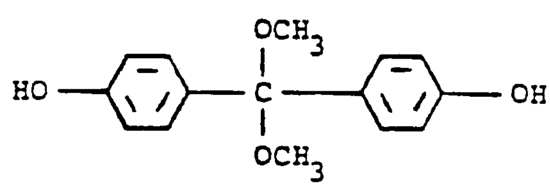

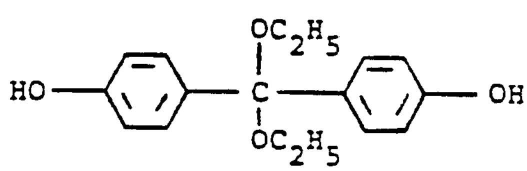

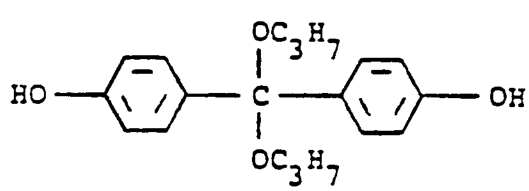

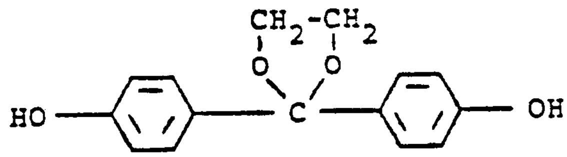

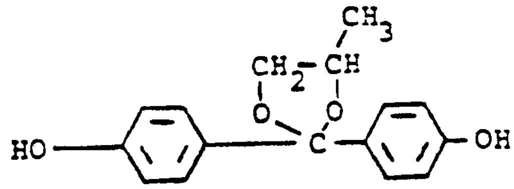

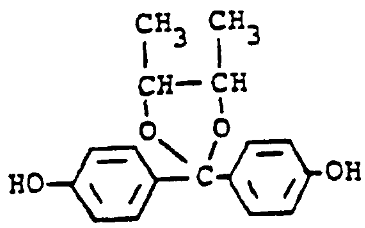

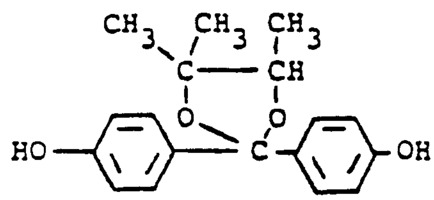

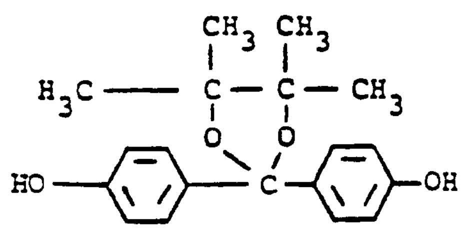

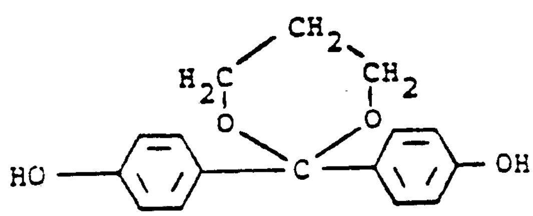

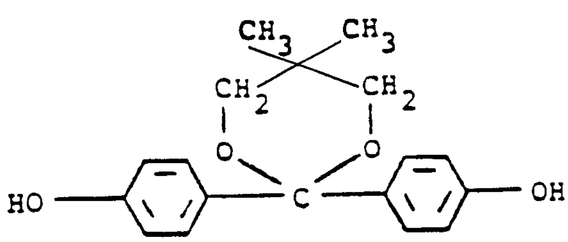

and their corresponding carboxylic acid esters.

The polyacetals are essentially linear polyethers comprised of the following repeat unit:

$$-\!\![O-W-O]_w-\!\![Z]_z-\!\![O-K-O]_K \qquad \text{(iii)}$$

where K, W and Z are as defined above in their general, preferred, and most preferred embodiments and

wherein k, w and z are the relative mole fractions selected so as to achieve the proper stoichiometric or near stoichiometric ratios for the desired oligomers and polymers. Thus it is obvious to one skilled in the art that the sum of k and w must closely approximate z.

Preferably, the mole fraction k is greater than or equal to 0.1.

Polymers of this invention are generally amorphous when w is small. It can be readily appreciated by one skilled in the art, however, that in those instances where k is nearly zero, i.e., by employing hardly any monomers HO—K—OH, and the resulting polymer is crystalline, then the formation of high molecular weight product is often more difficult to achieve due to crystallization of the polymer from the reaction medium. In such cases, it may be advantageous to use a sufficient proportion of HO—K—OH so as to maintain polymer solubility in the reaction medium and, in so doing, reduce or eliminate the crystallinity of the polymer.

The most preferred polymers are those of the aforementioned most preferred monomers, i.e., polymers containing the following structural repeat units:

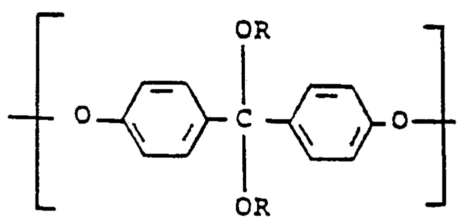

(v)

optionally with the following:

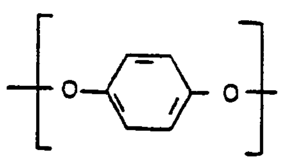

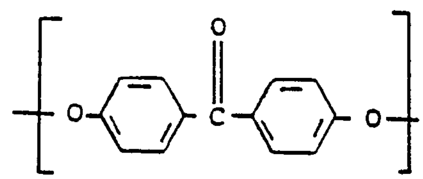

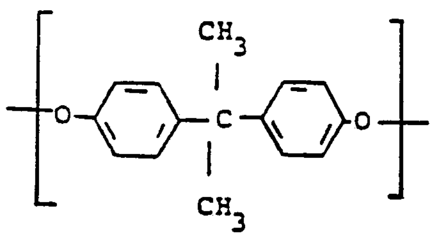

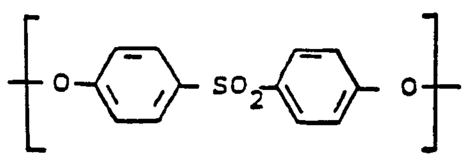

(vi)

together with the appropriate molar equivalent proportion of the following:

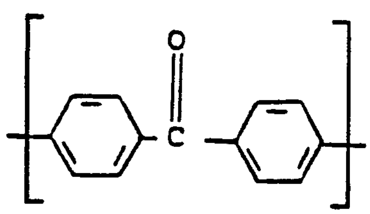

and/or

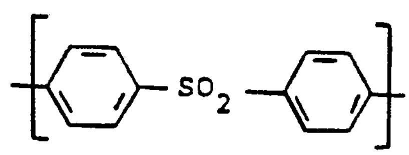

(vii)

where R is as defined above.

A preferred process for the preparation of the acetal monomers from the precursor diaromatic ketones containing at least one hydroxyl group ortho or para to the carbonyl comprises reacting the ketone precursor with a glycol in the presence of an alkylorthoester and a solid catalyst.

The precursor ketones are those analogous to the monomers HO—K—OH described herein except that the group —C(G)(G′) is replaced by a carbonyl and at least one hydroxyl group is situated ortho or para to said carbonyl.

The glycols, are of the general formula:

$$HO—CR''_2—E—CR''_2—OH$$

wherein R″ and E are as defined above, preferably with E being a single bond, and which include ethylene glycol, propylene glycol, 2,3-butanediol, 2-methyl-1,2-propanediol, 2-methyl-2,3-butanediol, 2,3-dimethyl-2,3-butanediol, 1,3-propanediol or 2,2,-dimethyl-1,3-propanediol.

The alkylorthoesters include trimethyl orthoformate, triethyl orthoformate, trimethyl orthoacetate, triethyl orthoacetate, tetramethyl orthosilicate or tetraethyl orthosilicate. Readily hydrolyzed compounds such as 2,2-dimethoxypropane or 2,2-dimethyl-1,3-dioxolane, which form volatile products such as methanol, ethanol or acetone, may be substituted for the orthoester.

The solid catalyst is preferably a finely divided acidic alumina-silica compound, and most preferably a montmorillonite clay as exemplified by the montmorillonite designated K—10 (obtained from United Catalysts). While the montmorillonite clays are preferred, other solid acidic catalysts with high surface areas may also function effectively as catalysts. These include e.g. acidic alumina or sulfonated polymer resins, as described in G. A. Olah et al, Synthesis, 282 (1981).

The reaction is conducted by simply mixing together the ketone precursor, about one equivalent or preferably an excess of the glycol, about one equivalent or preferably an excess of the orthoester, and at least 1 gram of the solid catalyst per equivalent of ketone, preferably 10 or more grams of solid catalyst per equivalent of ketone. The reaction is optionally conducted in the presence of an inert solvent. Since the catalyst is easily removed by filtration for reuse, large excesses of the solid may be conveniently employed.

The reaction is conducted at a temperature of from 25°C to the boiling point of the orthoester used, but preferably at a temperature below the boiling point of the orthoester but above the boiling point of the orthoester reaction products. For example a reaction temperature of from 65°C to 95°C is suitable when using trimethyl orthoformate (b.p. = 102°C) the reaction products of which are methanol (b.p. = 65°C) and methyl formate (b.p. = 34°C). Of course, the reaction temperature can be adjusted appropriately when conducting the reaction under reduced or elevated pressures.

The most preferred acetal monomer is preferably prepared by heating a mixture of 4,4'-dihydroxybenzophenone, excess glycol, excess trialkyl orthoformate, and 0.1 to 5 gm montmorillonite clay per gram of ketone and preferably from 0.5 to 2.5 grams of clay per gram of ketone, so as to distill off the alcohol derived from the orthoformate. The acetal, 2,2-bis(4-hydroxyphenyl)-1,3-dioxolane, can be obtained in excellent yield (60% to almost quantitative) in less than 48 hours reaction time.

Standard isolation methods can be employed to recover the acetal monomer and unreacted ketone, if any, with due care to avoid acidic aqueous environments. In some cases recrystallization or other extensive purification of the isolated reaction product may be unnecessary prior to use in the process to prepare a polyacetal. Thus, for example, after dilution of the reaction with ethyl acetate solvent, filtration to remove the solid catalyst, extraction of the solution with basic water to remove excess glycol, drying with a conventional drying agent such as anhydrous sodium sulfate, removal of the solvent and volatile materials under vacuum, and then washing the resulting solid with a solvent such as methylene chloride to remove minor contaminants, a reaction product is obtained which contains primarily acetal bisphenol monomer but may still contain some unreacted ketone precursor. This reaction product may be used without further purification to prepare high molecular weight polyacetal.

In general the reaction conditions employed to prepare the polyacetals are those used for effecting polymerization of bisphenols with bishalobenzenoid compounds for the preparation of polyarylethers.

According to the present invention the preparation of the polyacetals is conducted in the presence of a certain base in a certain dipolar aprotic solvent, and preferably in the presence of an inert azeotropic agent, at temperatures above about 100°C.

The base which is used is capable of reacting with the aromatic hydroxyls of the bisphenol monomers to form the mono or disalts thereof. According to the present invention, alkali metal carbonates, bicarbonates, hydroxides, and mixtures thereof, are used in near stoichiometric amounts or in excess. Altough the salts can often be formed separately and isolated for the polymerization reaction, it is usually preferable to react the hydroxyl monomers with the base in situ either prior to addition of the bishalobenzenoid monomer or during the polymerization step in the presence of the bishalobenzenoid monomer. In the latter case the alkali metal carbonates and mixtures thereof are particularly useful.

The dipolar aprotic solvents used according to the present invention are dialkylamides such as dimethylformamide and dimethylacetamide; cyclic alkylamides such as N-methylpyrrolidinone and N-propylpyrrolidinone, acyclic and cyclic ureas such as N,N'-dimethylpropyleneurea and 1,2-dimethyl-2-imidazolidinone; dialkyl and diaryl sulfoxides such as dimethyl sulfoxide; dialkyl, diaryl, and cyclic sulfones such as dimethyl sulfone, diphenyl sulfone, and sulfolane; and sulfamides and phosphoramides, such as N,N,N',N'-tetraethyl sulfamide and hexamethyl phosphoramide. Generally, the lower boiling solvents (b.p. $<$290°C) are preferred.

The azeotropic agent preferably used to remove the water of reaction or water introduced into the reaction is generally any inert compound which does not substantially interfere with the polymerization, codistills with water, and boils between 25° and 250°C. Common azeotropic agents include benzene, toluene, xylene, chlorobenzene, methylene chloride, dichlorobenzene, trichlorobenzene, and the like. It is advantageous, of course, to select the azeotropic agent such that its boiling point is less than that of the dipolar solvent used. Although an azeotropic agent is commonly used, it is not always necessary when higher reaction temperatures, for example, above 200°C, are employed especially when the reaction mixture is continuously sparged with inert gas.

It is generally desirable to conduct the reaction in the absence of oxygen under an inert atmosphere. The reaction can be carried out at atmospheric, subatmospheric, or superatmospheric pressures.

Other catalysts, salts, diluents, processing aids or additives may also be present or added during the

reaction provided they do not substantially interfere with the polymerization reaction, either directly or indirectly.

Reaction temperatures of up to about 250°C are generally sufficient for the polymerization reaction although higher temperatures can be used if necessary. The temperature will depend, of course, on the solvent boiling point and the reaction pressure and will also affect the reaction rate. In general, under atmospheric conditions, the reaction temperature will be from 100°C to 165°C in dimethylacetamide; to 240°C in sulfolane; and to 200°C in N-methylpyrrolidinone.

Obviously, the reaction solvent, the base, and the reaction temperature should be selected so as to obtain a reasonable polymerization rate and also to avoid degradation of the solvent, monomers or polymers which may cause interference with the polymerization. It is also preferable of course, to select the reaction solvent and reaction temperature so as to maintain the growing polymer chain in solution.

Once the desired polymer molecular weight is achieved, the phenate end groups can optionally be reacted by introducing an end-capping reagent, such as methyl chloride to form the stable methyl ether end group, or alternatively, reagents to form other reactive or stable end groups, as desired.

The preferred reaction conditions using the preferred monomers involve reacting, under argon or nitrogen atmosphere, essentially stoichiometric amounts of the monomers in the presence of from 1 to 50 percent excess of dried potassium carbonate in dimethylacetamide (or sulfolane) with toluene (or chlorobenzene) azeotrope at about 115°C (or 160°C) initially under reflux of the azeotropic solvent, gradually increasing the reaction temperature from 155 to 165°C (or from 180 to 220°C) by allowing some toluene (or chlorobenzene) to distill. The reaction is held at this temperature until the desired molecular weight polymer is formed, usually in 0.5 to 8 hours. The reaction mixture is diluted with dimethylacetamide (or sulfolane or other solvent) cooled to 100 to 150°C. Methyl chloride is then sparged through the reaction mixture for 0.2 to 2 hours to end-cap the polymer.

Commonly practiced polymer recovery methods can be used, such as coagulation into water or an organic (non)solvent; the recovered polymer is optionally washed with water and alcohol or other solvents and dried. Other recovery methods such as extraction, filtration, devolatilization, and the like, may also be used.

## Examples

The following examples serve to give specific illustrations of the practice of this invention.

The reduced viscosity (RV) of the polymer was measured in concentrated sulfuric acid at 25°C (1 gm of polymer dissolved in 100 ml of concentrated sulfuric acid). The calculation of the RV is based on the original polymer sample weight, regardless of any chemical reaction which may take place in the sulfuric acid solution. Therefore, the RVs are regarded as the RV in concentrated sulfuric acid solution (1 gm/100 ml solution) and not necessarily as the RV of the polymer itself.

### Example 1

Preparation of Dimethylacetal Bisphenol [bis(4-hydroxyphenyl)dimethoxymethane]

A reaction flask was charged with 11.0 grams (gm) of 4,4'-dihydroxybenzophenone (97 percent, 50 mmole), 6.5 gm of trimethylorthoformate (TMOF, 98 percent, 60 mmole), 75 ml methanol and 1 drop of concentrated aqueous hydrogen bromide and heated in an oil bath (100 to 106°C) with a reflux head set for very slow takeoff. After about 18 hours approximately 50 ml of material had distilled over; an additional 5 gm of TMOF, 50 ml of methanol, and one drop of hydrogen bromide was added and the reaction continued for another 6.5 hours with slow distillation. TMOF (4 gm), 25 ml of methanol, and one drop of hydrogen bromide was added and the reaction continued for 16 hours under total reflux. An additional 3 gm of TMOF and 15 ml of methanol was added and heating resumed with maximum takeoff for 7.5 hours by which time most of the methanol had distilled out. The reaction was cooled and neutralized with sodium acetate.

The residual solvent was removed under vacuum and the resulting orange residue was slurried in 200 ml of methylene chloride with 0.1 gm sodium carbonate. The slurry was filtered and the solid retreated with 200 ml of methylene chloride and refiltered. The combined methylene chloride solutions were evaporated to give 6.1 gm isolated product (after drying at 0.01 kPa (0.1 mm)). NMR analysis showed 90 percent of the acetal: an $A^2B^2$ quartet centered at 7.35 ppm (aromatic, 8H) and singlet at 3.20 ppm (methyls, 6H). Overall yield was 47 percent (correcting for purity).

The acetal was converted to its diacetate by reaction with acetic anhydride in pyridine (91 percent isolated yield). NMR of the diacetate showed the expected transitions: an $A^2B^2$ quartet at 7.23 ppm (aromatic, 8H), singlet at 3.07 ppm ($-OCH_3$, 6H), and singlet at 2.20 ppm ($CH_3CO-$, 6H).

### Example 2

2,2-bis(4-hydroxyphenyl)-1,3-dioxolane

*Azeotrope Method*

A 250 ml flask fitted with a magnetic stir bar, Dean-Stark trap, condenser, and drying tube was charged with 10 gm of 4,4'-dihydroxybenzophenone (46.7 mmoles), 16 gm of ethylene glycol (dried over molecular sieves, 258 mmole), 100 ml of benzene, and 2 drops concentrated aqueous hydrogen bromide and heated to reflux. After 4 days reflux, the trap was drained and charged with freshly dried molecular sieves and

fresh benzene, and reflux resumed. The molecular sieves in the trap were replaced with fresh sieves three times over the ensuing 13 days; in addition 2 more drops of hydrogen bromide and 100 ml of benzene were added during this time. Most of the benzene was distilled off and the reaction mixture dissolved in ethyl acetate, washed 4 times with 5% sodium bicarbonate solution, washed with sodium chloride solution and dried over sodium sulfate. The solvent was removed by evaporation under vacuum (final drying at 0.027 kPa (0.2 mm) pressure) to give crude product; NMR analysis showed a mixture of acetal and starting ketone. The crude material was slurried in 500 ml of methylene chloride, filtered and the methylene chloride solution evaporated to give essentially pure acetal; 3.7 gm (30.7% yield.) NMR spectrum of the acetal is consistent with the structure: $A^2B^2$ quartet centered at 7.45 ppm (aromatic, 8H), singlet at 4.07 ppm ($—OCH_2—$, 4H).

The diacetate of the acetal bisphenol was prepared by reaction with acetic anhydride in pyridine (75% yield). The NMR spectrum is also consistent: $A^2B^2$ quartet centered at 7.42 ppm (aromatic, 8H); singlet at 3.88 ppm ($—O—CH_2—$, 4H); and a singlet at 2.17 ppm ($CH_3CO—$, 6H). The melting point of the diacetate was 118°—121°C.

Elemental analysis of the acetal bisphenol gave 69.83% C, 5.59% H, 24.69% O; calculated 69.76% C, 5.46% H, and 24.78% O.

The preparation of 2,2-diphenyl-1,3-dioxolane from benzophenone under similar conditions is reported to give over 80% yield in only 5 hours reaction time, [M. Sulzbacher et al., J. Amer. Chem. Soc., 70, 2827 (1948)]. It is readily apparent that the synthesis of the acetal of 4,4'-dihydroxybenzophenone is accomplished less advantageously than the corresponding acetal of benzophenone.

Example 3

2,2-bis(4-hydroxyphenyl)-1,3-dioxolane

The procedure of Example 2 was repeated employing a very large excess of glycol and a much higher proportion of acid catalyst. Thus, 25 mmole of ketone, 625 mmole of ethylene glycol, and 2.5 mmole of hydrogen bromide were reacted under benzene azeotrope (80°C); after an extended reaction time, i.e. 5 days, NMR analysis of the reaction mixture showed 80% conversion of the ketone to the desired acetal.

Example 4

2,2-bis(4-hydroxyphenyl)-4-methyl-1,3-dioxolane

*Azeotrope Method*

The procedure of Example 3 was repeated replacing the ethylene glycol with propylene glycol and the benzene with toluene. Thus, 50 mmole of ketone, 1250 mmole of propylene glycol [1,2-propanediol], and 2.4 mmole of hydrogen bromide catalyst were heated under toluene reflux (113°C); after 4 days reaction time, NMR analysis of the reaction mixture showed 90% conversion to the desired acetal.

Example 5

2,2-bis(4-hydroxyphenyl)-1,3-dioxolane

*Clay/Orthoformate Method*

A one-liter flask fitted with a mechanical stirrer, jacketed condenser, and a variable take-off distillation head was charged with 66 gm of 4,4'-dihydroxybenzophenone (97%, 0.30 mmole), 186 gm of ethylene glycol (3 moles), 96 gm of trimethylorthoformate (0.9 mole), and 150 gm acidic montmorillonite clay (K—10, United Catalysts). The reaction mixture was stirred and heated in an oil bath (75 to 80°C) for 18 hours while distilling over methylformate and methanol. An additional 96 gm of trimethylorthoformate was added and heating continued for 25 hours. A sample was taken from the reactor and NMR analysis showed 82% conversion to acetal. An additional 36 gm of trimethylorthoformate was added and the reaction mixture heated in a bath (100 to 110°C) until distillation had essentially stopped.

The reaction mixture was cooled, diluted with ethyl acetate, filtered to remove the clay, and the clay washed with ethyl acetate. The organic solution was washed four times with 2% solution of sodium bicarbonate, once with saturated sodium chloride solution, dried over sodium sulfate, filtered, and the solvent removed under vacuum. The crude product was slurried with 200 ml of methylene chloride, filtered, and dried to give 57.6 gm of product. Gas chromatographic analysis of the acetylated product (acetic anhydride, pyridine) showed it to contain 86.6% of the desired acetal and 13.4% starting 4,4'-dihydroxybenzophenone. The conversion based on isolated product was 64.8%; the total isolated yield was 76.9% including recovered ketone. Compared to Examples 2 and 3 high yields are obtained in significantly shorter reaction times.

Example 6

The procedure of Example 5 was repeated using triethylorthoformate instead of trimethylorthoformate and at higher reaction temperature. Thus, 10 mmoles of ketone, 50 mmoles of ethylene glycol and 30 mmoles of triethylorthoformate in the presence of 1 gm of montmorillonite clay were reacted at 120°C for 24 hours. Isolation of the reaction product gave 2.48 gm which was shown to be 77.4% pure by gpc; the calculated conversion to acetal was 74%. Thus, high yield was obtained.

Example 7

The procedure of Example 5 was repeated except that azeotropic reflux with benzene was used instead of the orthoformate. Thus 75 mmoles of ketone, 750 mmoles of ethylene glycol, and 15 gm of montmorillonite clay were refluxed with 75 ml of benzene at 80°C using a Dean-Stark trap to collect the water distilled over; after 47 hours the reaction was worked up yielding 14.3 gm crude product the NMR analysis of which showed 25 mole% acetal (18% conversion). This example illustrates that the clay catalyst does not effect efficient formation of the acetal when benzene azeotrope instead of orthoester is used to remove the water of reaction.

Example 8

The procedure of Example 5 was repeated except that hydrogen bromide catalyst was used instead of the clay. Thus, 60 mmoles of ketone, 340 mmoles of ethylene glycol, and a total of 170 mmoles of trimethylorthoformate added in portions were reacted in the presence of 3 drops concentrated hydrogen bromide at 85°C for a total of 120 hours; toward the end of this period the reaction temperature was raised to 120°C to distill off the excess unreacted orthoformate; work up of the reaction yielded 13.1 gm of product which was shown to contain about 21 mole% acetal by NMR (calculated conversion 17.8%). A similar experiment using hydrogen bromide and p-toluenesulfonic acid together in place of the clay gave an isolated product containing only 36 mmole% acetal after 120 hours at 70—95°C.

This example illustrates that using strong acid catalyst in place of the clay does not effect efficient conversion to the acetal even when orthoester is present.

Example 9

Preparation of Polyacetal

A 500 ml 4-neck reaction flask fitted with a mechanical stirrer, thermometer, argon inlet, jacketed Vigreux column, Dean-Stark trap and condenser was charged with 23.16 gm of 2,2-bis(4-hydroxyphenyl)-1,3-dioxolane (97.95 percent acetal and 2.05 percent of 4,4'-dihydroxybenzophenone by vpc analysis, 90.03 mmoles total monomers), 19.64 gm of 4,4'-difluorobenzophenone (90.03 mmole), 160 ml of dried dimethylacetamide, 115 ml of toluene, and 18.68 gm of dried, anhydrous potassium carbonate. The reaction mixture was stirred and purged with argon for one hour, heated to reflux in an oil bath, and the reflux temperature was gradually increased from 119° to 150°C by removing distillate from the trap and adding small amounts of toluene to the reaction flask. After about 5.5 hours, a solution of 0.02 gm of 4,4'-difluorobenzophenone in 2 ml of dimethylacetamide was added to the viscous reaction mixture to assure stoichiometry. After an additional 30 minutes, the heating bath was removed and 135 ml dimethylacetamide added to dilute the reaction mixture.

The reaction temperature was then adjusted to 110°C and methyl chloride gas was bubbled through the reaction mixture for about one hour (using the argon inlet tube) to end-cap the phenate end-groups, during which the yellow-green reaction mixture changed to a creamy beige color. The reaction mixture was then heated to 150°C and filtered through a sintered glass funnel. The filtrate was coagulated into excess isopropanol and the polymer washed with isopropanol, distilled water, and methanol and dried under vacuum at 100°C to give 35.5 gm of polymer (89.8 percent isolated yield). The RV of the polymer was 0.80 in chloroform (0.2 percent, 25°C) and 1.64 in concentrated sulfuric acid (1 percent, 25°C).

The polymer was molded at 250°C to give a clear, tough plaque with excellent color with the following mechanical properties:

Tensile modulus (ASTM D—638) (280,000 psi) 1930 MPa
Tensile strength (ASTM D—638) (9,520 psi) 65.6 MPa
Yield strength (ASTM D—638) (8,800 psi) 60.6 MPa
Yield elongation (ASTM D—638) 5.0%
Elongation at break (ASTM D—638) 115%
Pendulum impact strength (ASTM D—256) >(250ft-lbs/$in^3$) 20.7 $Nm/cm^3$
Glass transition temperature 155°C

The polymer was amorphous, i.e., exhibited no melting transition by differential scanning calorimetry.

Example 10

Preparation of Polyacetal

The reaction of 2,2-bis(4-hydroxyphenyl)-1,3-dioxolane (4.655 gm, 18.03 mmole), 4,4'-dihydroxybenzophenone (0.429 gm, 2.00 mmole) and 4,4'-difluorobenzophenone (4.370 gm, 20.03 mmole) was conducted by the procedure of Example 9 except on a smaller scale (4.15 gm potassium carbonate, 35 ml dimethylacetamide, 25 ml toluene charged) to give polyacetal polymer with an RV equal to 1.37 (1 percent in sulfuric acid at 25°C).

This example shows that 4,4'-dihydroxybenzophenone can be substituted for at least 10 mole percent of the ketone monomer and high molecular weight polymer produced.

Example 11

Polyacetal was prepared by the procedure of Example 10 except that the bisphenol monomer mixture was the reaction product prepared and isolated essentially as described in Example 5. Thus, the reaction

was charged with 25.2235 gm of 2,2-bis(4-hydroxyphenyl)-1,3-dioxolane reaction product containing 13.72% unreacted 4,4'-dihydroxybenzophenone by gpc analysis (0.1 mole total bisphenols by gpc analysis), 22.1473 gm of 4,4'-difluorobenzophenone (0.1015 mole), 125 ml of dimethylacetamide, 125 ml of toluene and 20.75 gm of potassium carbonate. The polymerization was conducted as in Example 9; after 5 hours at 150 to 160°C, the polymer was end-capped with methyl chloride and recovered by coagulation yielding 37.6 gm. of polymer with an RV = 1.17 (one percent in concentrated sulfuric acid, 25°C).

This example illustrates that high molecular weight polyacetal polymer was prepared using the isolated reaction product obtained from the improved acetal process as in Example 5 without requiring extensive monomer purification such as recrystallization.

Example 12

2,2-bis(4-hydroxyphenyl)-1,3-dioxolane was prepared by the procedure of Example 5 by mixing together, in a reaction flask fitted with a mechanical stirrer, thermometer, and variable take-off distillation head, 99 gm of 4,4'-dihydroxybenzophenone (97% pure, 0.448 mole), 269 gm of ethylene glycol (4.3 moles), 96 gm of trimethyl orthoformate (0.91 mole), and 150 gm of montmorillonite clay (K—10, United Catalysts) and heating the reaction mixture at 70 to 90° to give slow distillation of the reaction byproducts. After about 18 hours, 66 gm of distillate had been collected, an additional 64 gm of trimethylorthoformate (0.60 mole) was added to the reaction mixture, and the reaction continued. After a total of 24 hours reaction time, NMR analysis of a reaction sample showed about 2.23 mole ratio of acetal product to ketone starting material; after a total of 48 hours reaction time, NMR analysis of a second reaction sample showed the mole ratio was about 19 (about 95% conversion to acetal product). The reaction mixture was heated for an additional 8 hours and then cooled; NMR analysis again showed about 95% conversion.

The reaction mixture was worked up as in Example 5 by dilution with ethyl acetate, filtration to remove the clay, extraction with basic water to remove glycol, drying over anhydrous sodium sulfate, and removal of solvent to give 115 gm of crude product which was then ground, stirred twice with methylene chloride, filtered, and the solid dried under vacuum to give 99.9 gm of creamy white product. Gas chromatographic analysis of the derivatized diacetate product (acetic anhydride, pyridine) showed it to contain 95.4 wt% acetal and 4.6% ketone. The isolated yield of acetal was 82.5% (87.2% yield including recovered ketone).

This example illustrates that the just described process affords as much as 95% conversion to 2,2-bis(4-hydroxyphenyl)-1,3-dioxolane within 48 hours and excellent isolated yields of this acetal bisphenol can be obtained.

**Claims**

1. A process for preparing a polyacetal polymer which comprises reacting
(a) monomers of the following formulae:

HO—K—OH

wherein K has up to 40 carbon atoms and is selected from one or more of the following:

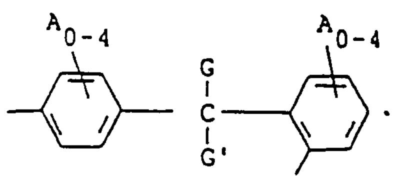
.
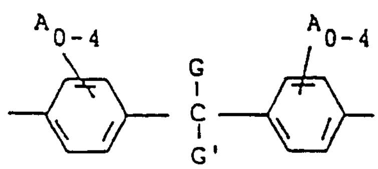

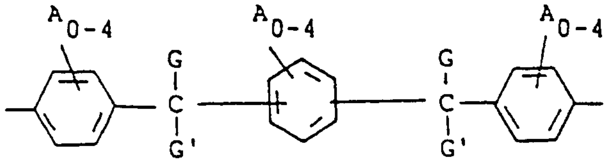

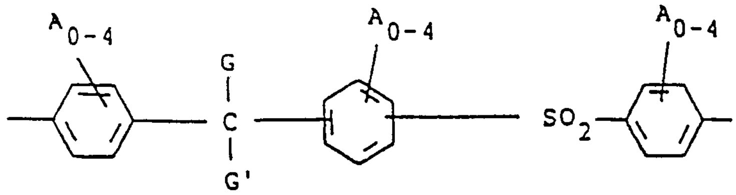

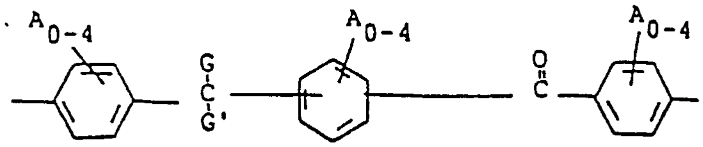

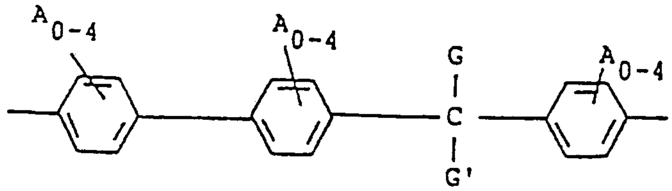

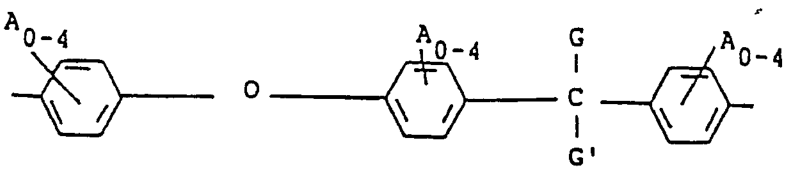

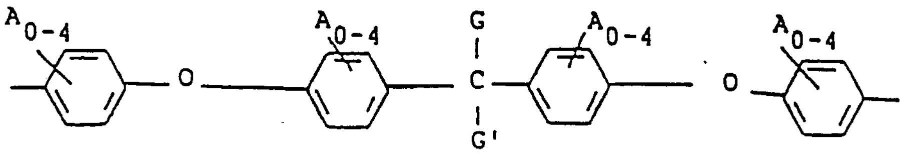

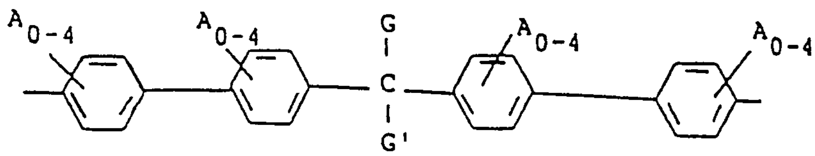

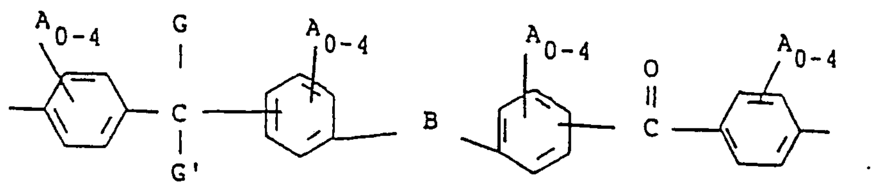

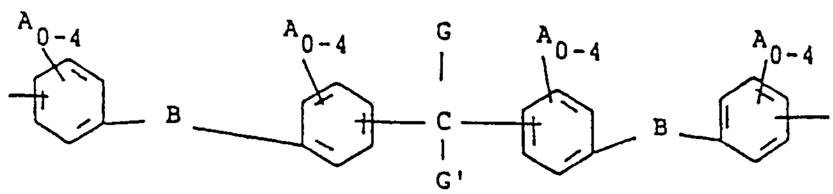

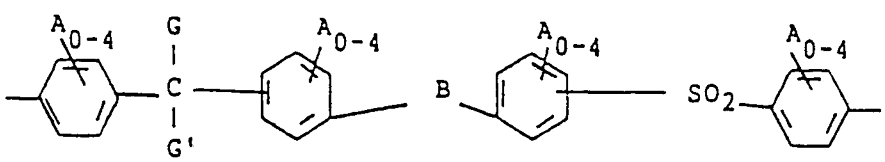

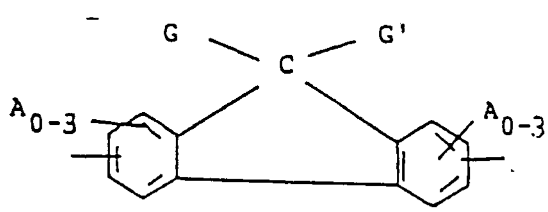

wherein A is independently selected from alkyl, aryl, halogen and cyano, which are non-interfering under the reaction conditions, B is an electron withdrawing group in ortho- or para-position, selected from —$SO_2$—, —CO—, —SO—, or a single bond, —O—, —S—, —S—S— or a difunctional hydrocarbon radical of from 1 to 20 carbon atoms and G and G′ may be the same or different and represent —OR, wherein the groups R are each independently alkyl, aryl or arylalkyl of from 1 to 20 carbon atoms, may be substituted or

unsubstituted, may contain heteroatoms and may also be connected by a chemical bond thus connecting G and G', with the proviso that the R's should not contain functionality which is base sensitive; with

(b) one or more monomers X—Z—Y, where Z is

$$-Ar^1-Q-\left[Ar^2-Q'\right]_n-Ar^3-$$

or

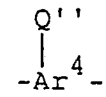

wherein the Ar's are substituted or unsubstituted aryl radicals of up to 18 carbon atoms, n is 0 to 3, Q and Q' are electron withdrawing groups ortho or para to X and Y and selected from $—SO_2—$, —CO—, —SO—, and Q'' is an electron withdrawing group ortho or para to X and Y and selected from $—NO_2$, —CN, or $—SO_mR'$, R' being a hydrocarbon group, and m being 1 or 2, and wherein X and Y are halogen, —OSOR' or $—OSO_2R'$;

(c) optionally one or more bisphenols HO—W—OH, where W is selected from the following:

$$—Ar^1—Q—\left[Ar^2—Q'\right]_n—Ar^3—,\quad —Ar^4—\quad \text{and}\quad —Ar^5—V—Ar^6—,$$

where n, the Ar's, Q and Q' are as defined above, and wherein V is a single bond, —O—, —S—, —S—S— or a difunctional hydrocarbon radical of from 1 to 20 carbon atoms; in the presence of a base in near stoichiometric amounts or in excess, said base being selected from alkali metal carbonates, bicarbonates, hydroxides, and mixtures thereof, in a dipolar aprotic solvent selected from dialkylamides, cyclic alkylamides, acyclic and cyclic ureas, dialkyl and diaryl sulfoxides, dialkyl, diaryl and cyclic sulfones, sulfamides and phosphoramides at temperatures above 100°C.

2. Process according to claim 1 wherein K is of the following formulae:

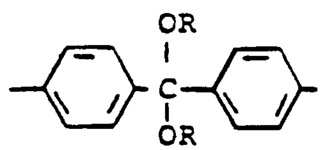

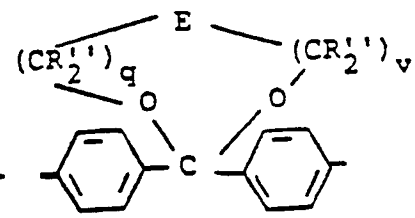

wherein R is defined as in claim 1 and R'' is independently selected from hydrogen, alkyl, aryl and arylalkyl containing from 1 to 20 carbon atoms, E is selected from a single bond, double bond, difunctional hydrocarbon, carbonyl, —O—, —S—, —SO—, $—SO_2—$, —NR—, and difunctional silicon, q is 1 or 2, and v is 1 or 2.

3. Process according to any one of claims 1 and 2 wherein the monomers (a) are selected from:

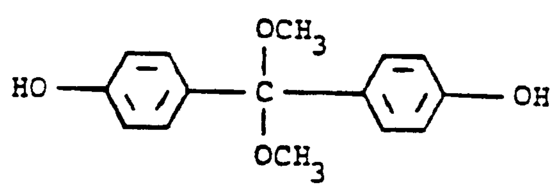

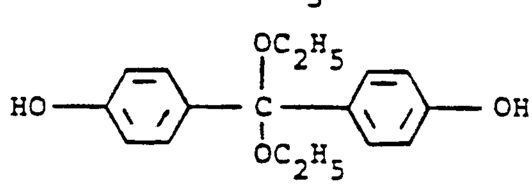

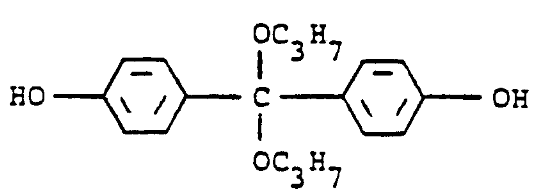

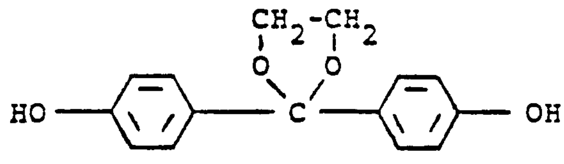

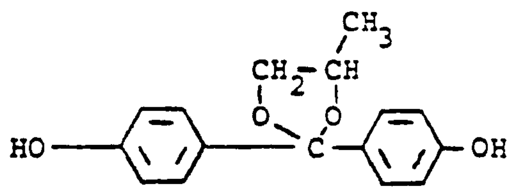

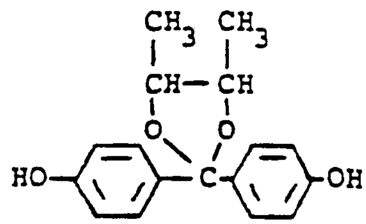

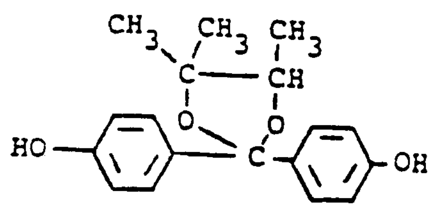

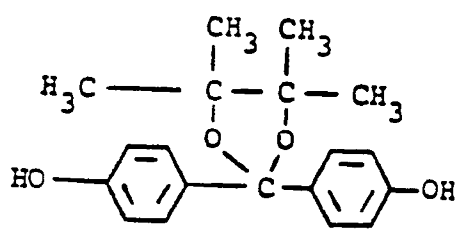

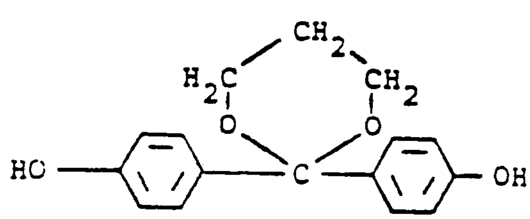

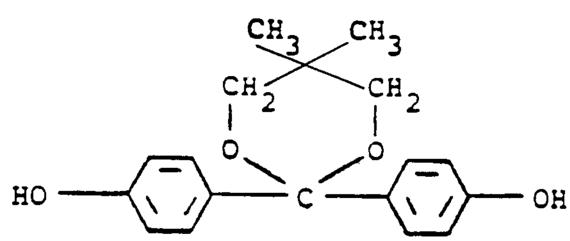

and the carboxylic acid esters thereof.

4. A compound of the following formula:

HO—K—OH

wherein K is defined as in claim 1.

5. A compound according to claim 4 wherein K is defined as in claim 2.

6. A compound according to any one of claims 4 and 5 of the following formulae:

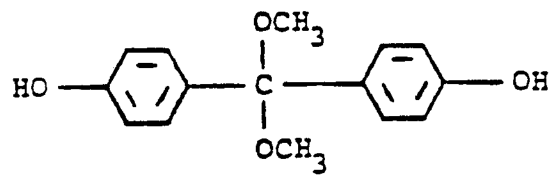

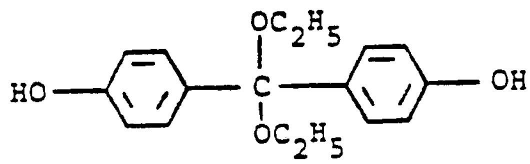

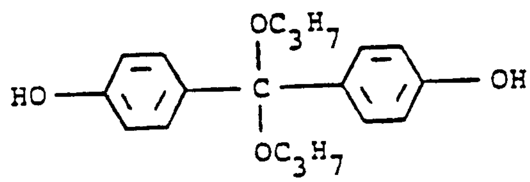

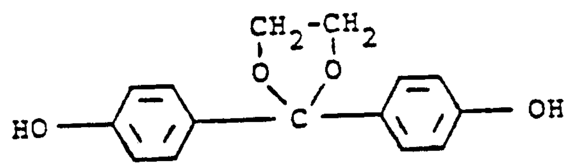

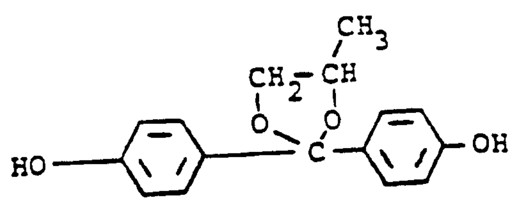

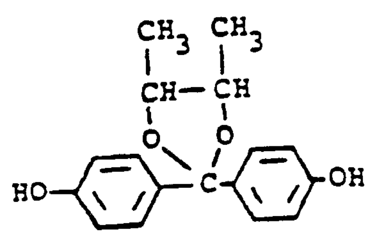

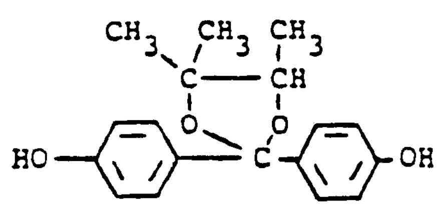

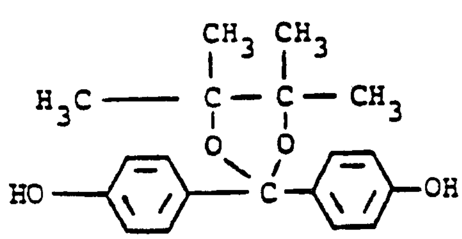

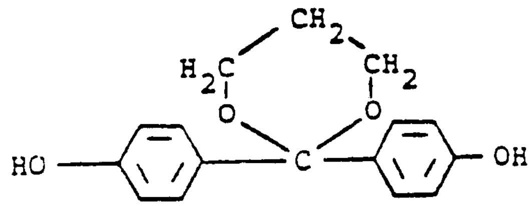

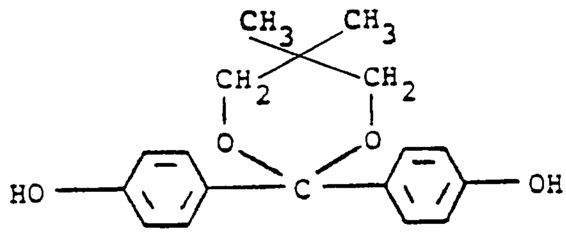

**Patentansprüche**

1. Verfahren zur Herstellung eines Polyacetal-Polymers, welches umfaßt die Umsetzung von (a) Monomeren der folgenden Formeln:

HO—K—OH

worin K bis zu 40 Kohlenstoffatomen hat und aus einem oder mehreren der Folgenden ausgewählt ist:

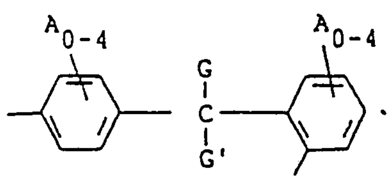

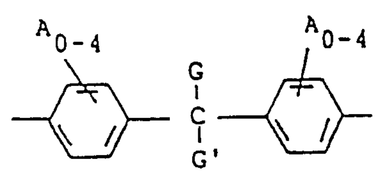

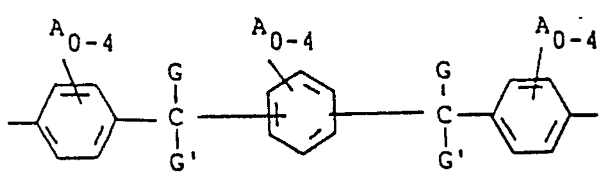

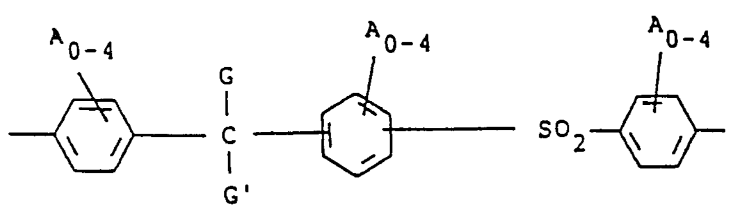

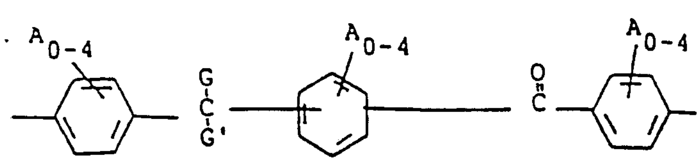

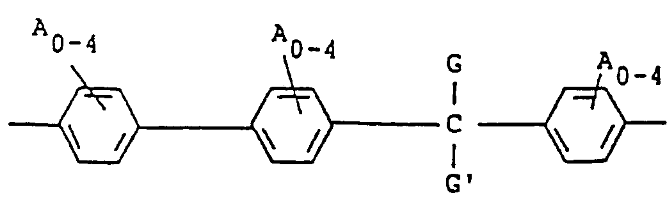

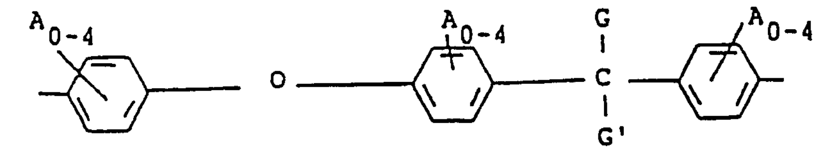

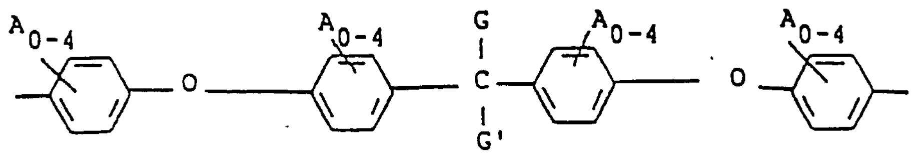

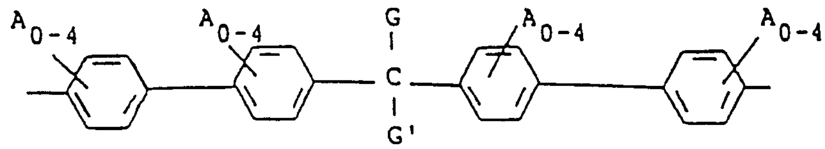

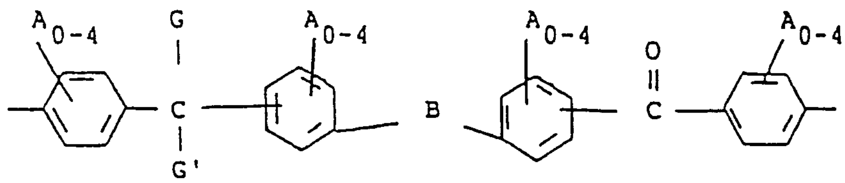

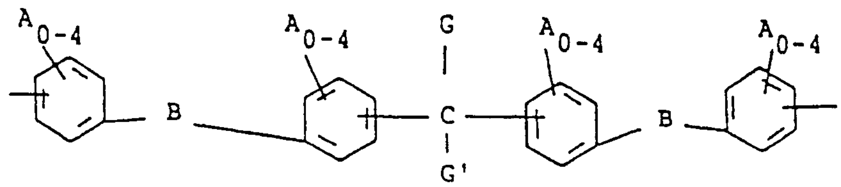

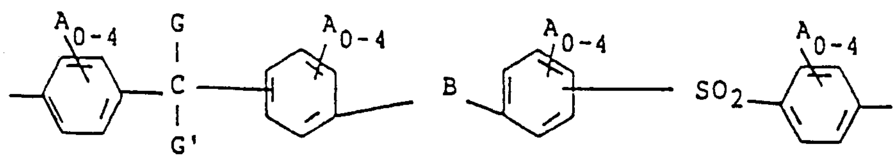

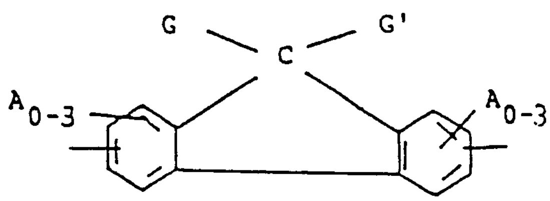

worin A unabhängig aus Alkyl, Aryl, Halogen und Cyano, welche unter den Reaktionsbedingungen nicht stören, ausgewählt ist, B eine elektronenabziehende Gruppe in o- oder p-Stellung, ausgewählt aus $—SO_2—$, —CO—, —SO—, oder eine Einfachbindung, —O—, —S—, —S—S— oder ein bifunktioneller Kohlenwasserstoffrest mit 1 bis 20 Kohlenstoffatomen ist, und G und G' gleich oder verschieden sein können und repräsentieren —OR, worin die Gruppen R jeweils unabhängig voneinander Alkyl, Aryl oder Arylalkyl mit 1 bis 20 Kohlenstoffatomen bedeuten, substituiert oder unsubstituiert sein können, Heteroatome enthalten können, und auch durch eine chemische Bindung miteinander verbunden sein können, auf diese Weise G und G' verbindend, mit der Maßgabe, daß die Reste R keine basenempfindliche Runktionalität enthalten sollten; mit

(b) einem oder mehreren Monomeren X—Z—Y, worin Z

$$-Ar^1-Q-\left[Ar^2-Q'\right]_n-Ar^3-$$ oder 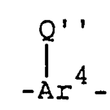

bedeutet, worin die Ar's substituierte oder unsubstituierte Arylreste mit bis zu 18 Kohlenstoffatomen sind,

n 0 bis 3 ist, Q und Q' elektronenabziehende Gruppen ortho oder para zu X und Y und aus —$SO_2$—, —CO—, und —SO— ausgewählt sind, und Q'' eine elektronenabziehende Gruppe ortho oder para zu X und Y und aus —$NO_2$, —CN oder —$SO_mR'$ ausgewählt ist, wobei R' eine Kohlenwasserstoffgruppe darstellt und m 1 oder 2 ist, und worin X und Y Halogen, —OSOR' oder —$OSO_2R'$ sind;

(c) gegebenenfalls einem oder mehreren Bisphenolen HO—W—OH, worin W aus den Folgenden ausgewählt ist:

$$-Ar^1-Q-\left[Ar^2-Q'\right]_n-Ar^3-, \quad -Ar^4- \quad \text{und} \quad -Ar^5-V-Ar^6-,$$

worin n, die Ar's, Q und Q' wie oben definiert sind, und worin V eine Einfachbindung —O—, —S—, —S—S— oder einen bifunktionellen Kohlenwasserstoffrest mit 1 bis 20 Kohlenstoffatomen darstellt; in der Gegenwart einer Base in annähernd stöchiometrischen Mengen oder im Überschuß, wobei die Base aus Alkalimetallcarbonaten, -bicarbonaten, -hydroxiden, und Mischungen davon, ausgewählt ist, in einem dipolaren aprotischen Lösungsmittel, ausgewählt aus Dialkylamiden, cyclischen Alkylamiden, acyclischen und cyclischen Harnstoffen, Dialkyl- und Diarylsulfoxiden, Dialkyl-, Diaryl- und cyclischen Sulfonen, Sulfamiden und Phosphoramiden bei Temperaturen über 100°C.

2. Verfahren nach Anspruch 1, worin K eine der folgenden Formeln hat:

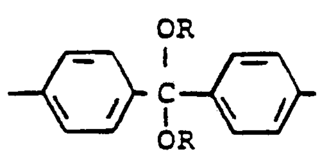

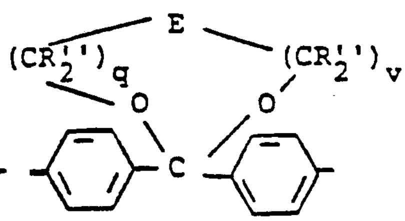

worin R wie in Anspruch 1 definiert ist und R'' unabhängig aus Wasserstoff, Alkyl, Aryl und Arylalkyl mit 1 bis 20 Kohlenstoffatomen ausgewählt ist, E aus einer Einfachbindung, einer Doppelbindung, einem bifunktionellen Kohlenwasserstoff, Carbonyl, —O—, —S—, —SO—, —$SO_2$—, —NR— und bifunktionellem Silizium ausgewählt ist, q 1 oder 2 ist und v 1 oder 2 ist.

3. Verfahren nach irgendeinem der Ansprüche 1 und 2, worin die Monomeren (a) ausgewählt sind aus:

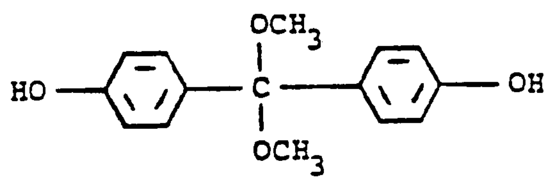

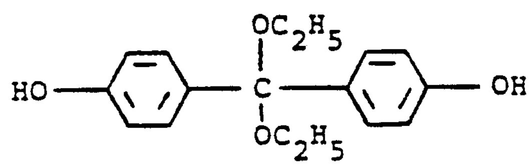

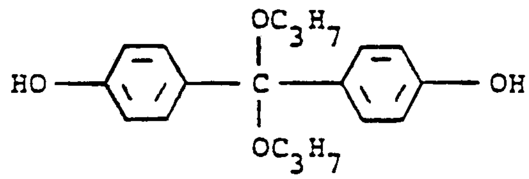

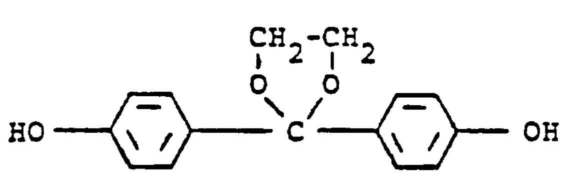

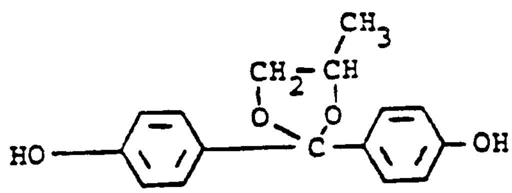

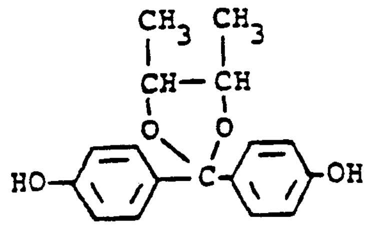

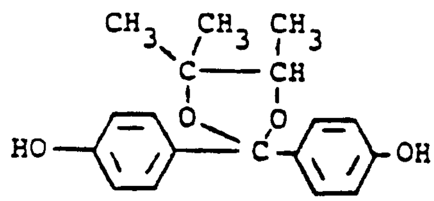

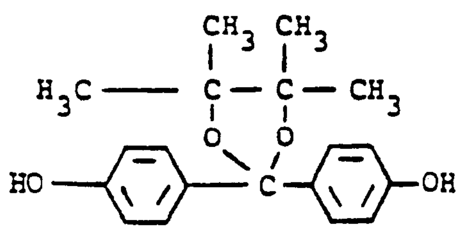

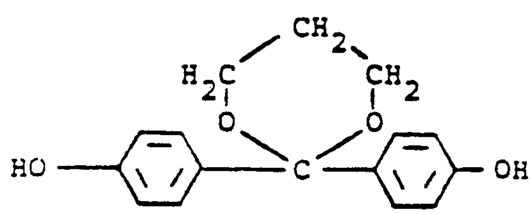

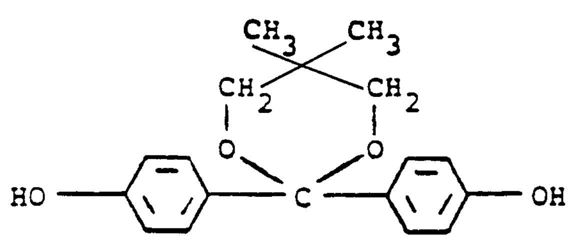

und deren Carbonsäureestern.

4. Eine Verbindung der folgenden Formel:

HO—K—OH

worin K wie in Anspruch 1 definiert ist.

5. Eine Verbindung nach Anspruch 4, worin K wie in Anspruch 2 definiert ist.

6. Eine Verbindung nach irgendeinem der Ansprüche 4 und 5 mit einer der folgenden Formeln:

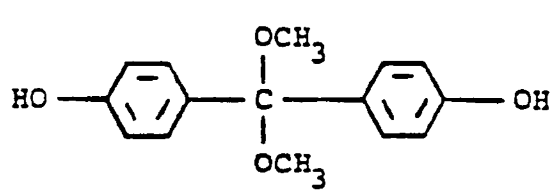

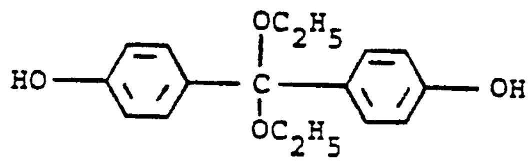

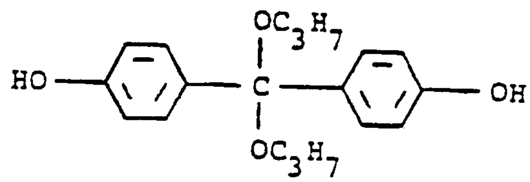

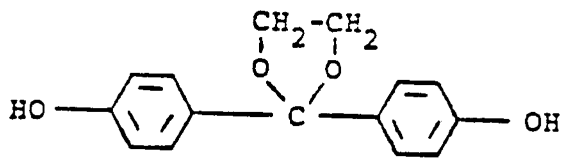

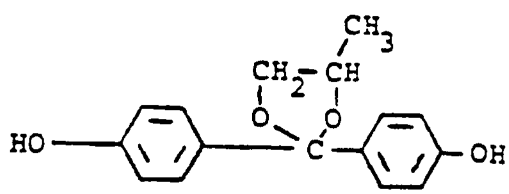

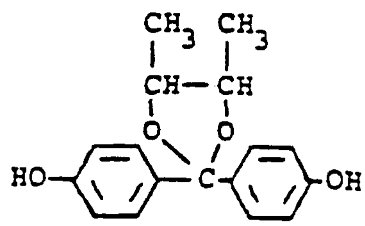

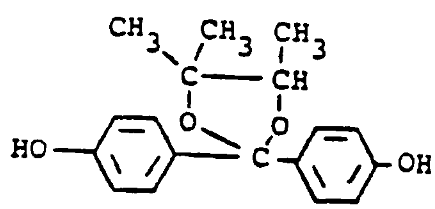

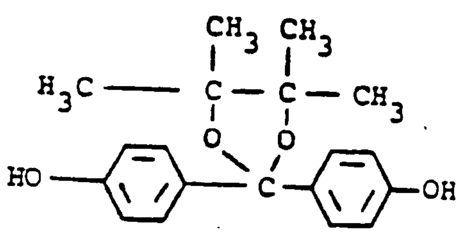

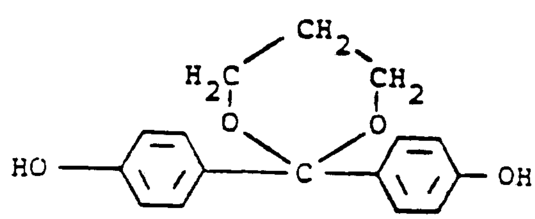

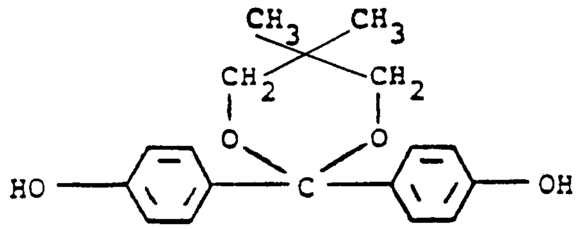

## Revendications

1. Procédé de préparation d'un polymère du type polyacétal, qui consiste à faire réagir (a) des monomères répondant aux formules suivantes:

HO—K—OH

où K possède jusqu'à 40 atomes de carbone et est choisi entre un ou plusieurs des groupes suivants:

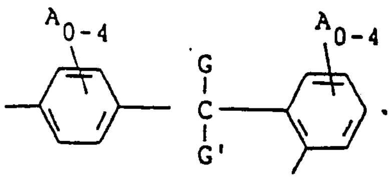

,

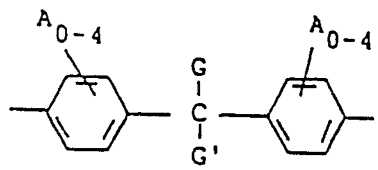

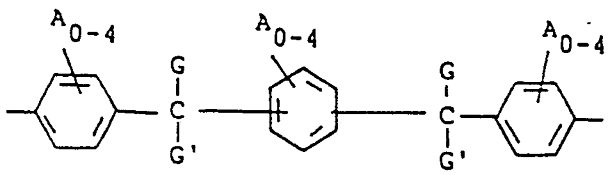

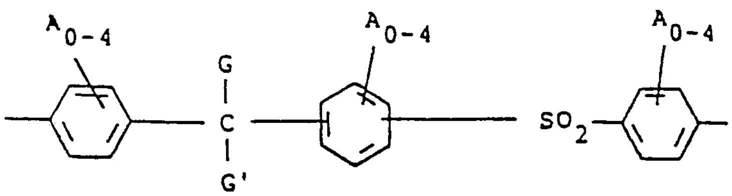

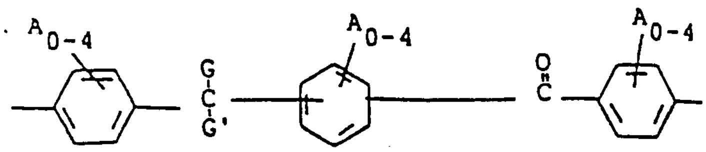

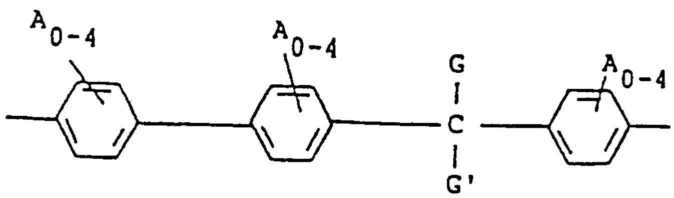

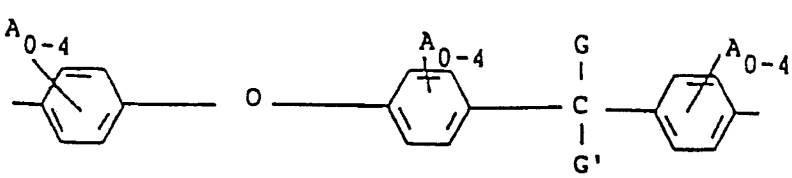

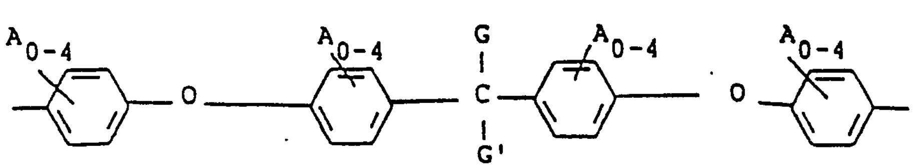

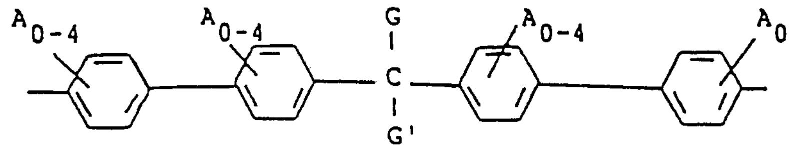

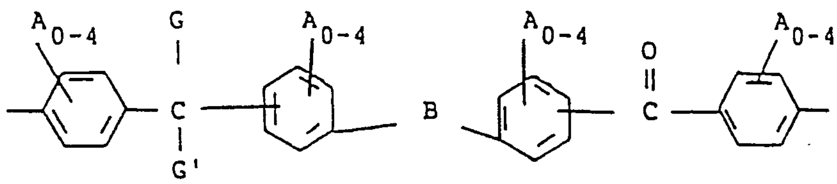

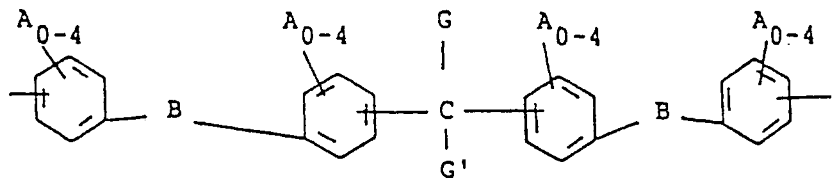

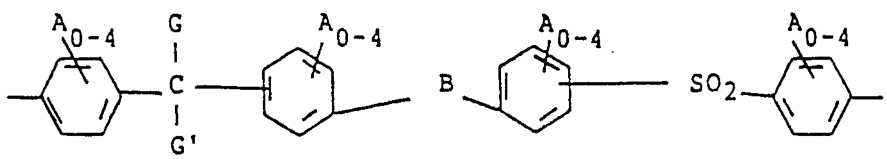

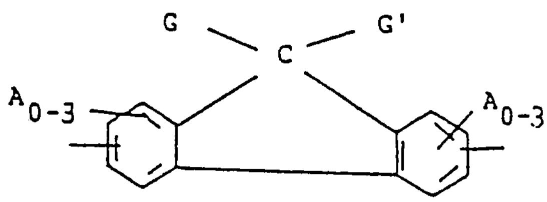

dans lesquels A est choisi indépendamment entre des groupes alkyle, aryle, halogéno et cyano, qui sont non interférents dans les conditions réactionnelles, B est un groupe électrophile en position ortho ou para, choisi entre $—SO_2—$, —CO—, —SO—, ou une liaison simple, —O—, —S—, —S—S— ou un radical hydrocarboné difonctionnel ayant 1 à 20 atomes de carbone, et G et G′ peuvent être identiques ou différents et représentent un groupe de formule —OR, dans laquelle les groupes R représentent chacun indépendamment des groupes alkyle, aryle ou arylalkyle ayant 1 à 20 atomes de carbone, peuvent être substitués ou non substitués, peuvent contenir des hétéroatomes et peuvent également être reliés par une liaison chimique, assurant ainsi la connexion de G et G′, sous réserve que les groupes R ne contiennent pas une fonctionnalité qui soit sensible aux bases; avec

(b) un ou plusieurs monomères de formule X—Z—Y, dans laquelle Z représente un groupe

$$-Ar^1-Q-\left[Ar^2-Q'\right]_n-Ar^3- \quad \text{ou} \quad \underset{-Ar^4-}{\overset{Q''}{|}}$$

dans lequel les groupes Ar sont des radicaux aryle substitués ou non substitués ayant jusqu'à 18 atomes de carbone, n a une valeur de 0 à 3, Q et Q′ sont des groupes électrophiles en position ortho ou para par rapport à X et Y et choisis entre $—SO_2—$, —CO— et —SO—, et Q″ représente un groupe électrophile en position ortho ou para par rapport à X et Y, qui est choisi entre $—NO_2$, —CN— et $—SO_mR'$, R′ représentant un groupe hydrocarboné et m étant égal à 1 ou 2, et dans laquelle X et Y représentent un halogène, un groupe —OSOR′ ou $—OSO_2R'$;

(c) facultativement, un ou plusieurs bisphénols de formule HO—W—OH, dans laquelle W est choisi entre les groupes suivants:

$$—Ar^1—Q—\left[Ar^2—Q'\right]_n—Ar^3—, \quad —Ar^4— \quad \text{et} \quad —Ar^5—V—Ar^6—,$$

dans lesquels n, les groupes Ar, Q et Q' répondent aux définitions précitées, V représente une liaison simple, —O—, —S—, —S—S— ou un radical hydrocarboné difonctionnel ayant 1 à 20 atomes de carbone; en présence d'une base en des quantités pratiquement stoechiométriques ou en excès, ladite base étant choisie entre des carbonates, des bicarbonates, des hydroxydes de métaux alcalins et leurs mélanges, dans un solvant aprotique dipolaire choisi entre des dialkylamides, des alkylamides cycliques, des urées acycliques et cycliques, des dialkyl- et diaryl-sulfoxydes, des dialkyl- et diaryl-sulfones ainsi que des sulfones cycliques, des sulfamides et des phosphoramides, à des températures supérieures à 100°C.

2. Procédé suivant la revendication 1, dans lequel K est choisi entre des groupes répondant aux formules suivantes:

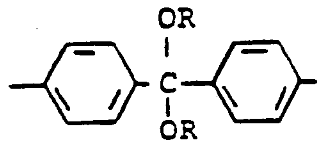

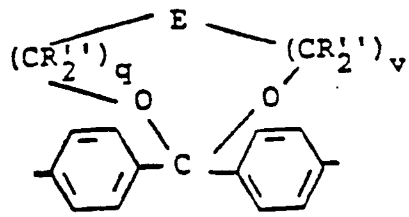

dans lesquelles R répond à la définition suivant la revendication 1 et R'' est choisi indépendamment entre l'hydrogène, des groupes alkyle, aryle et arylalkyle contenant 1 à 20 atomes de carbone, E est choisi entre une liaison simple, une double liaison, un radical hydrocarboné difonctionnel, carbonyle, —O—, —S—, —SO—, —$SO_2$—, —NR— et un silicium difonctionnel, q est égal à 1 ou 2, et v est égal à 1 ou 2.

3. Procédé suivant l'une quelconque des revendications 1 et 2, dans lequel les monomères (a) sont choisis entre:

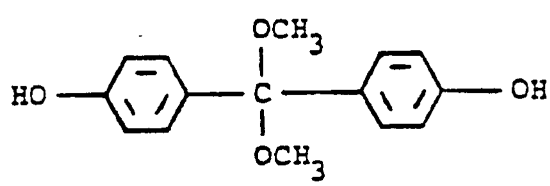

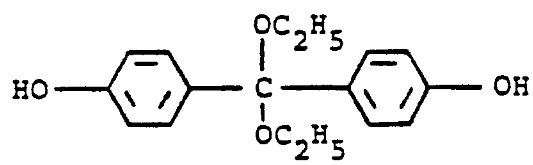

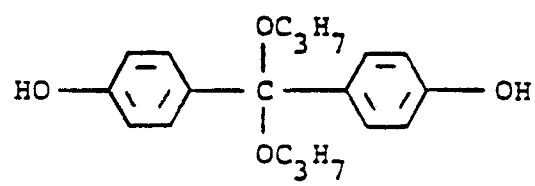

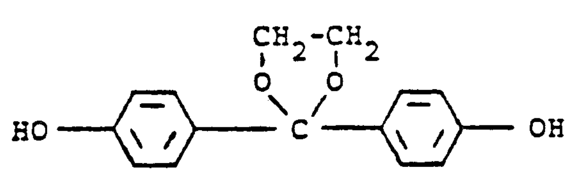

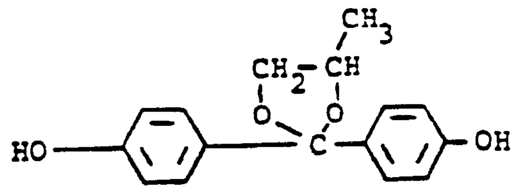

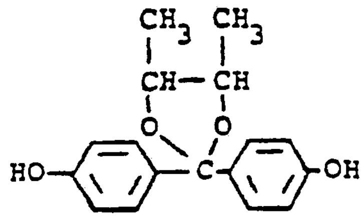

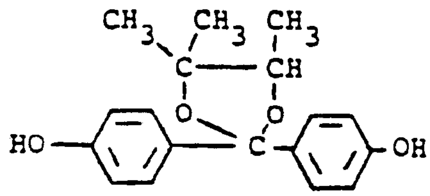

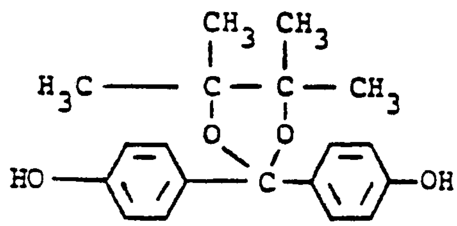

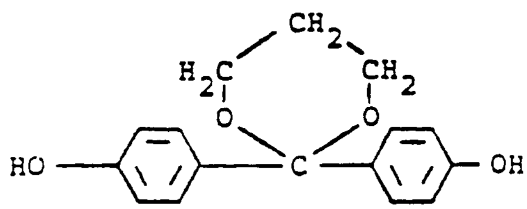

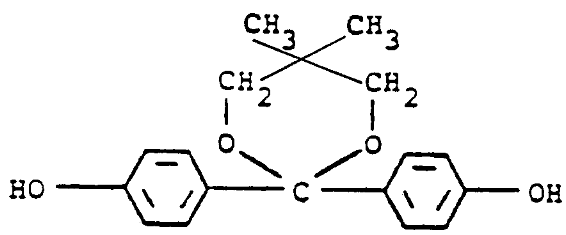

et leurs esters d'acides carboxyliques.

4. Composé répondant à la formule suivante:

$$HO—K—OH$$

dans laquelle K répond à la définition suivant la revendication 1.

5. Composé suivant la revendication 4, dans lequel K répond à la définition suivant la revendication 2.

6. Composé suivant l'une quelconque des revendications 4 et 5, répondant à l'une des formules suivantes: